# EUROPEAN PATENT SPECIFICATION

(11) **EP 4 101 401 B1**
(45) Date of publication and mention of the grant of the patent: **04.09.2024**
(21) Application number: 22178126.3
(22) Date of filing: 09.06.2022
(51) Int. Cl.: A61B 17/29

(54) **ELECTROSURGICAL INSTRUMENTS**
ELEKTROCHIRURGISCHE INSTRUMENTE
INSTRUMENTS ÉLECTROCHIRURGICAUX

(30) Priority: 10.06.2021 US 202163209088 P; 10.05.2022 US 202217740505
(43) Date of publication of application: 14.12.2022
(73) Proprietor: Covidien LP, Mansfield, MA 02048 (US)
(72) Inventor: MOUA, Tony, G, Boulder, 80301 (US); JOSEPH, Daniel, A, Boulder, 80301 (US)
(74) Representative: Maschio & Soames IP Ltd

(56) References cited:
- EP-A1- 3 476 333
- EP-B1- 3 216 403
- CN-A- 107 019 537

## Description

### CROSS-REFERENCE TO RELATED APPLICATION

This application claims the benefit of and priority to U.S. Provisional Patent Application No. 63/209,088, filed June 10, 2021.

### FIELD

The present disclosure relates generally to surgical instruments. In particular, the disclosure relates to shaft-based surgical instruments such as electrosurgical forceps.

### BACKGROUND

Instruments such as electrosurgical forceps are commonly used in open and endoscopic surgical procedures to coagulate, cauterize and/or seal tissue. Such forceps typically include a pair of jaw members that can be controlled by a surgeon to grasp targeted tissue, such as, e.g., a blood vessel. The jaw members may be approximated to apply a mechanical clamping force to the tissue, and are associated with at least one electrode to permit the delivery of electrosurgical energy to the tissue. The combination of the mechanical clamping force and the electrosurgical energy has been demonstrated to join adjacent layers of tissue captured between the jaw members to form a tissue seal.

A bipolar electrosurgical forceps typically includes electrodes disposed on the opposed jaw members. The electrodes are charged to opposite electrical potentials such that an electrosurgical current may be selectively transferred through tissue grasped between the electrodes. The electrosurgical forceps is typically equipped with a mechanism that allows a clinician to manually rotate the jaw members without having to rotate the entire instrument. Documents CN107019537 A, EP3476333 A1 and EP3216403 B1 disclose instruments of the prior art.

### SUMMARY

Claim 1 defines the invention and dependent claims disclose embodiments. No surgical methods are claimed. The techniques of this disclosure generally relate to a gear system incorporated into a surgical instrument (e.g., a shaft-based electrosurgical forceps) that reduces the amount of rotation of an actuator needed to rotate an end effector a selected amount. As such, a clinician may rotate the end effector a selected amount by rotating the actuator to a lesser degree than the selected amount. The gear system may include planetary gears, worm gears, bevel gears, helical gears, or other suitable gear mechanisms. In aspects, a selective gear ratio mechanism (e.g., a clutch) may be provided to allow a clinician to select different actuation input to output rotation ratios. A ratchet-type rotation wheel or lever may be implemented to allow ratcheting motion to rotate the end effector. In other aspects, a motor-driven system may be provided for smoother and easier function. In aspects, one actuator may be actuated to rotate the end effector clockwise, and another actuator may be actuated to rotate the end effector counter-clockwise; alternatively, single-direction rotation may be provided, e.g., in 90 degree, 180 degree, or other suitable increments.

In one aspect of the present disclosure, provided is a surgical instrument including a handle assembly and a shaft portion. The handle assembly includes a handle housing, an actuator rotatably coupled to the handle housing, and a main gear operably coupled to the actuator. The shaft portion has a proximal end portion rotatably supported by the handle assembly, and a distal end portion configured to support an end effector. The shaft portion is configured to rotate relative to the handle assembly about a longitudinal axis defined by the shaft portion. The shaft portion is non-rotatably coupled to the main gear and is configured to rotate with the main gear in response to a rotation of the actuator at a rate greater than a rate of rotation of the actuator.

In aspects, the handle assembly may further include a ring gear rotationally fixed to the handle housing, and a plurality of planet gears rotationally supported on the actuator and in meshing engagement with the ring gear. The main gear may be a sun gear in meshing engagement with the planet gears such that the rotation of the actuator rotates the planet gears relative to the ring gear to rotate the sun gear.

In aspects, the sun gear may be fixed to the proximal end portion of the shaft portion.

In aspects, the actuator may be configured to slide relative to the sun gear between a first position and a second position. In the first position, the actuator is engaged to the planet gears. In the second position, the actuator is engaged directly to the main gear.

In aspects, the actuator may be a rotation wheel that protrudes outwardly from the handle housing.

In aspects, the rotation wheel may define a plane that is parallel with the longitudinal axis of the shaft portion.

In aspects, the rotation wheel may be configured to rotate about a rotation axis that is perpendicular to the longitudinal axis of the shaft portion.

In aspects, the rotation wheel may have a first annular array of teeth in meshing engagement with the main gear.

In aspects, the rotation wheel may have an upper surface, and the first annular array of teeth may extend upwardly relative to the upper surface.

In aspects, the first annular array of teeth may be disposed at a radial distance from the rotation axis of the rotation wheel that is at least double a diameter of the main gear.

In aspects, a gear ratio between the rotation wheel and the main gear may be from 1:5 to 1:2.

In aspects, the rotation wheel may have a second annular array of teeth disposed at a different radial distance from the rotation axis than the first annular array of teeth.

In aspects, the main gear may be configured for selective engagement with the first or second annular array of teeth to adjust a gear ratio between the rotation wheel and the main gear.

In aspects, the actuator may be a lever arm.

In accordance with another aspect of the disclosure, a surgical instrument is provided that includes a handle assembly and a shaft portion. The handle assembly includes a handle housing, a rotation wheel rotationally supported in the handle housing, a ring gear rotationally fixed to the handle housing, a plurality of planet gears rotationally supported on the rotation wheel and in meshing engagement with the ring gear, and a sun gear in meshing engagement the planet gears such that a rotation of the rotation wheel in a first direction rotates the plurality of planet gears relative to the ring gear to rotate the sun gear. The shaft portion may have a proximal end portion rotatably supported by the handle assembly, and a distal end portion configured to support an end effector. The shaft portion is configured to rotate relative to the handle assembly about a longitudinal axis defined by the shaft portion. The shaft portion is non-rotatably coupled to the sun gear such that the shaft portion is configured to rotate in the first direction with the sun gear in response to a rotation of the rotation wheel in the first direction.

In aspects, the rotation wheel may be configured to slide relative to the sun gear between a first position and a second position. In the first position, the rotation wheel is engaged to the planet gears. In the second position, the rotation wheel is engaged directly to the sun gear.

In aspects, a gear ratio between the rotation wheel and the sun gear may be from 1:5 to 1:2.

As is traditional, the term "distal" refers herein to an end of the electrosurgical instrument or component thereof that is farther from an operator, and the term "proximal" refers herein to the end of the electrosurgical forceps or component thereof that is closer to the operator.

### BRIEF DESCRIPTION OF THE DRAWINGS

Objects and features of the present disclosure will become apparent to those of ordinary skill in the art when descriptions of various embodiments thereof are read with reference to the accompanying drawings, of which:
FIG. 1 is a perspective view, with a handle housing half removed, of an electrosurgical forceps according to the invention including a handle assembly, a shaft portion, and an end effector;
FIG. 2 is an enlarged perspective view illustrating a gear assembly of the handle assembly of FIG. 1, in accordance with the invention;
FIG. 3 is a perspective view of the gear assembly of FIG. 2;
FIG. 4 is a perspective view of a rotation wheel of the gear assembly of FIG. 2;
FIG. 5 is a rear, perspective view, with the rotation wheel removed, of the gear assembly of FIG. 2;
FIG. 6A is a side, perspective view of an electrosurgical forceps illustrating a lever arm in a first state according to an aspect of the present disclosure;
FIG. 6B is a side, perspective view of the electrosurgical forceps of FIG. 6A illustrating the lever arm in a second state;
FIG. 7 is a perspective view of an electrosurgical forceps according to another aspect of the present disclosure including a handle assembly, a shaft portion, and an end effector;
FIG. 8 is a perspective view, with a handle housing half removed, of the electrosurgical forceps of FIG. 7;
FIG. 9 is an enlarged perspective view illustrating a gear assembly of the handle assembly of FIG. 8; and
FIG. 10 is a side, perspective view of an electrosurgical forceps according to another aspect of the present disclosure.

### DETAILED DESCRIPTION

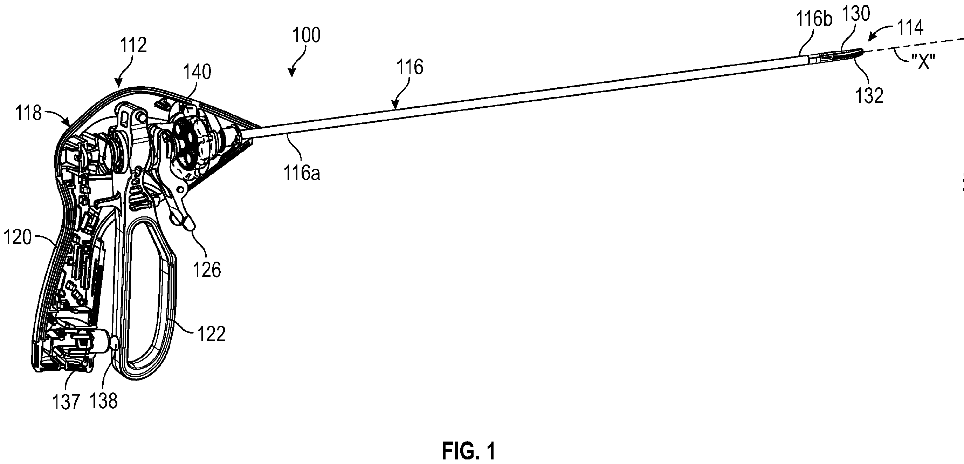

Referring initially to FIG. 1, a surgical instrument, such as, for example, an electrosurgical forceps 100 is provided and generally includes a handle assembly 112 that supports various actuators thereon for remotely controlling an end effector 114 through a shaft portion 116. Although this configuration is typically associated with instruments for use in laparoscopic or endoscopic surgical procedures, various aspects of the present disclosure may be practiced with traditional open instruments and in connection with endoluminal procedures as well. The handle assembly 112 includes a handle housing 118 constructed of a left housing half and a right housing half. The housing halves may be constructed of sturdy plastic, and may be joined to one another by adhesives, ultrasonic welding, mechanical fastening, or other suitable assembly methods. The shaft portion 116 has a proximal end portion 116a rotatably supported in the handle housing 118, and a distal end portion 116b configured to support the end effector 114.

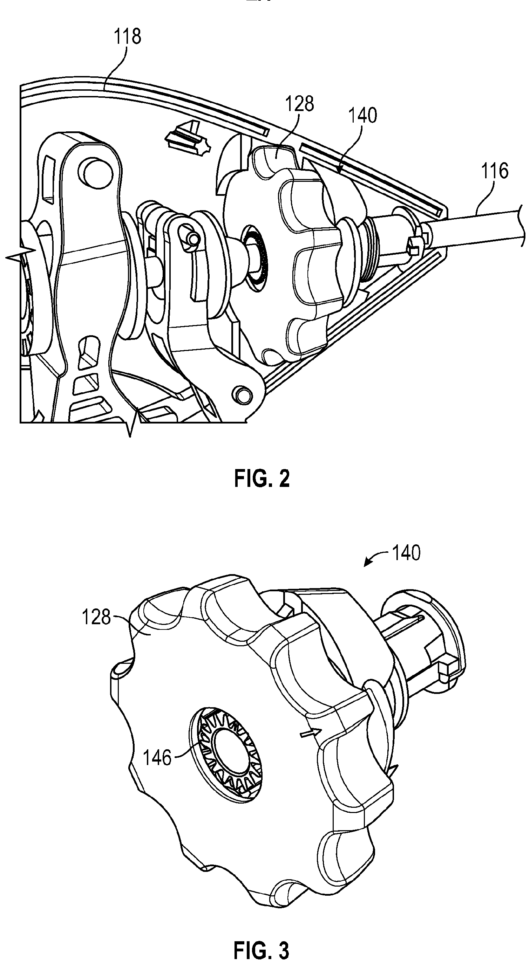

To mechanically control the end effector 114, the housing 118 supports a stationary handle 120, a movable handle 122, a trigger 126, and an actuator, such as, for example, a rotation knob or wheel 128 (FIG. 2). The present disclosure also contemplates robotic implementations wherein the handle housing mounts on a robotic arm and the manual actuators are replaced with robotic inputs. Thus, terms like handle, lever, etc. that are typically associated with manual grasping and/or manipulation are utilized herein to also cover robotic, powered, or other non-manual implementations. The movable handle 122 is operable to move the end effector 114 between an open configuration wherein upper and lower jaw members 130, 132 are disposed in spaced relation to one another, and a closed or clamping configuration wherein the jaw members 130, 132 are closer together, e.g., for grasping tissue. Approximation of the movable handle 122 with the stationary handle 120 moves the end effector 114 to the closed configuration and separation of the movable handle 122 from the stationary handle 120 moves the end effector 114 to the open configuration. The trigger 126 is operable to extend and retract a knife blade (not explicitly shown) through the end effector 114 when the end effector 114 is in the closed configuration. The rotation wheel 128 (FIG. 2) rotates the shaft portion 116 and the end effector 114 about a longitudinal axis "X" defined by the shaft portion 116, as will be described in further detail.

To electrically control the end effector 114, the stationary handle 120 supports a depressible button 137 thereon, which is operable by the user to initiate and terminate the delivery of electrosurgical energy to the end effector 114. The depressible button 137 is mechanically coupled to a switch (not shown) disposed within the stationary handle 120 and is engageable by a button activation post 138 extending from a proximal side of the moveable handle 122 upon proximal movement of the moveable handle 122 to an actuated or proximal position. The switch is in electrical communication with an electrosurgical generator (not explicitly shown) via suitable electrical wiring extending from the housing 118 through a cable extending between the housing 118 and the electrosurgical generator. The cable may include a connector (not shown) thereon such that the forceps 100 may be selectively coupled electrically to the generator. Further details about the end effector 114 may be found, for example, in U.S. Patent No. 8,298,232, entitled "ENDOSCOPIC VESSEL SEALER AND DIVIDER FOR LARGE TISSUE STRUCTURES".

With reference to FIGS. 2-5, the handle assembly 112 further includes a gear assembly 140 supported in the handle housing 118 and configured to transmit rotational motion originating in the rotation wheel 128 to the shaft portion 116 and the attached end effector 114. The gear assembly 140 may be a planetary gear assembly including a ring gear 142, a plurality of planet gears 144, and an elongated main gear or sun gear 146. The ring gear 142 is rotationally fixed to the handle housing 118 and defines a plurality of gear teeth 148 extending inwardly. The planet gears 144 are rotationally supported on the rotation wheel 128 and in meshing engagement with the ring gear 142 and the sun gear 146. In aspects, the gear assembly 140 may include four planet gears. In other aspects, the gear assembly 140 may include more or less than four planet gears. The sun gear 146 extends centrally through the ring gear 142 and is in meshing engagement with each of the planet gears 144 such that a rotation of the rotation wheel 128 rotates the planet gears 144 relative to the ring gear 142 to rotate the sun gear 146.

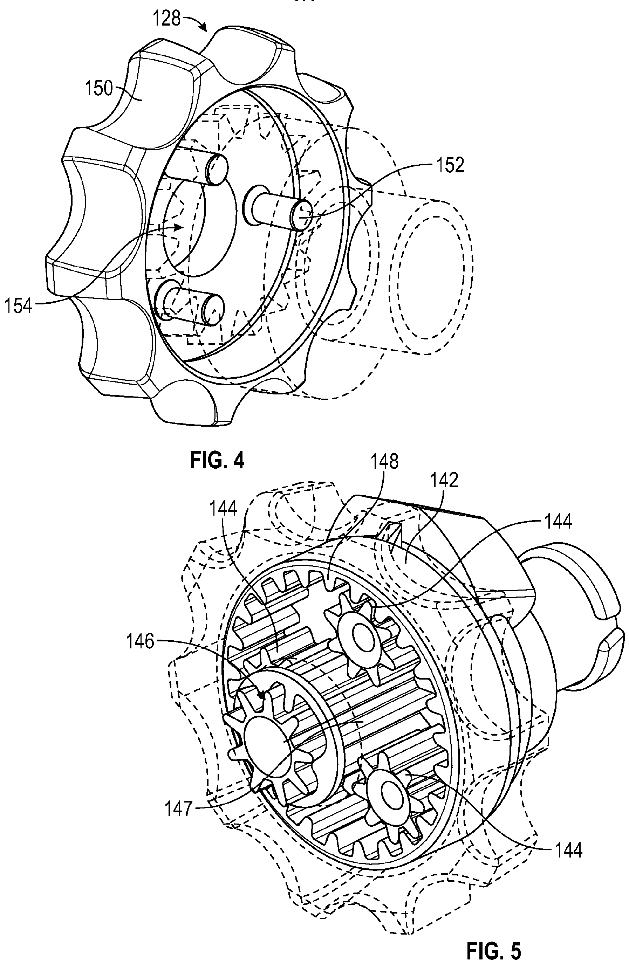

As best shown in FIG. 4, the rotation wheel 128 functions as a planet carrier and includes an annular body portion 150 that protrudes outwardly through slots defined within the handle housing 118 on either side thereof and a plurality of pegs 152 protruding longitudinally from the annular body portion 150. The pegs 152 are configured for receipt in the corresponding planet gears 144 and permit rotation of the planet gears 144 thereabout. The annular body portion 150 of the rotation wheel 128 defines a channel 154 therethrough configured for receipt of the sun gear 146.

The gear ratio between the rotation wheel 128 and the sun gear 146 may range from about 1:2 to about 1:5. As such, for every degree of rotation inputted by the rotation wheel 128, there is a greater degree of rotation output by the sun gear 146 and, in turn, the shaft portion 116 and the attached end effector 114. The sun gear 146 may have from about 4 teeth to about 10 teeth, and in some aspects 8 teeth; each of the four planet gears 144 may have between about 4 teeth to about 10 teeth, and in some aspects 8 teeth; and the ring gear 142 may have from about 18 teeth to about 35 teeth, and in some aspects 25 teeth.

With continued reference to FIGS. 2 and 5, the proximal end portion 116a of the shaft portion 116 has the sun gear 146 non-rotatably fixed thereto such that the shaft portion 116 is configured to rotate with the sun gear 146 in response to a rotation of the rotation wheel 128. For example, the sun gear 146 may have a detent (not explicitly shown) protruding inwardly therefrom that is received in a corresponding recess (not explicitly shown) defined in the proximal end portion 116a of the shaft portion 116 to non-rotatably couple the sun gear 146 to the shaft portion 116. In aspects, the sun gear 146 may be fixed to the shaft portion 116 via other suitable fastening engagements, such as, for example, monolithic formation, adhesive, fasteners, or the like.

In aspects, the channel 154 defined in the rotation wheel 128 may allow for translational movement of the rotation wheel 128 relative to and along the sun gear 146. The rotation wheel 128 may have a plurality of teeth (not explicitly shown) protruding into the channel 154 of the annular body portion 150. The teeth are configured for meshing engagement with gear teeth 147 of the sun gear 146 when the rotation wheel 128 is moved to a proximal position. More specifically, the rotation wheel 128 may be configured as a clutch that slides relative to and along the sun gear 146 between a distal or first position, in which the pegs 152 of the rotation wheel 128 are received in the planet gears 144 to indirectly couple the rotation wheel 128 to the sun gear 146, and the proximal or second position, in which the pegs 152 of the rotation wheel 128 are withdrawn from the planet gears 144 and the teeth of the rotation wheel 128 directly engage with the gear teeth 147 of the sun gear 146. In this aspect, a clinician may selectively move the rotation wheel 128 between the first and second positions to adjust the gear ratio between the rotation wheel 128 and the shaft portion 116. In such aspects, plural gear assemblies 140 may be provided, each having a different gear ratio and being selectively engagable with rotation wheel 128. In aspects, a pin (not shown) may be provided that may be used to selectively lock the rotation wheel 128 to the sun gear 146 such that the sun gear 146 is configured to rotate with the rotation wheel 128 in a 1:1 gear ratio.

In aspects, the gear ratio associated with gear assembly 140 may be selected such that a maximum rotation of rotation wheel 128 with a finger of the clinician without the need for repositioning the clinician's finger on rotation wheel 128 (e.g., wherein rotation wheel 128 is engaged with the clinician's finger at one end of the slot defined through handle housing 118 and is rotated to the opposite end of the slot defined through handle housing 118) corresponds to a rotation of the shaft portion 116 and the attached end effector 114 of about 90 degrees or about 180 degrees.

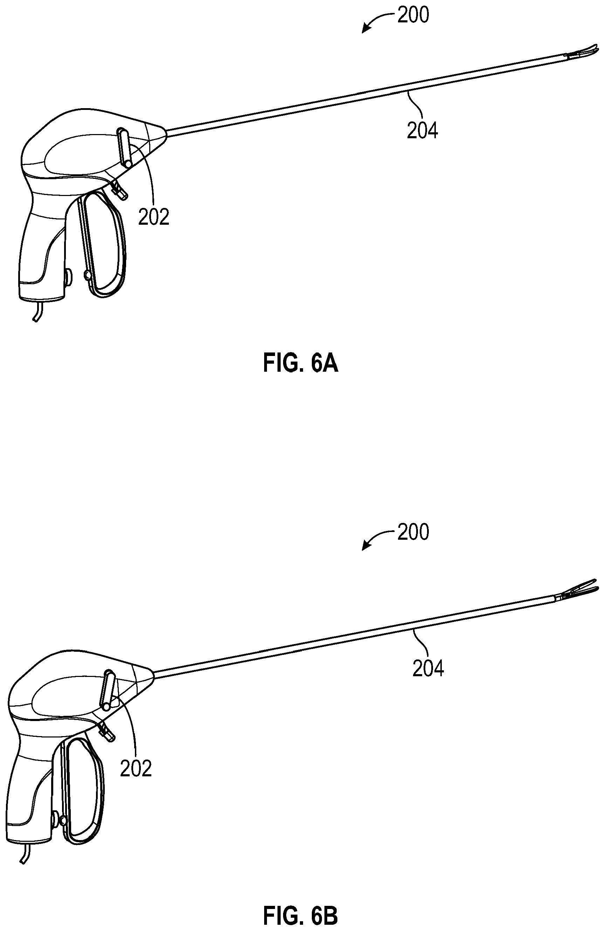

FIGS. 6A and 6B illustrate an electrosurgical forceps 200 similar to electrosurgical forceps 100 except that the rotation wheel 128 is replaced with a lever arm 202. The lever arm 202 is configured to rotate a gear system (not explicitly shown), such as, for example, a worm drive, that rotates the shaft portion 204 at a greater rate of rotation than the lever arm 202, or a gear system similar to gear assembly 140 (FIG. 3). For example, a rotation of the lever arm 202 from a starting position (FIG. 6A) to a second position (FIG. 6B) of about 30 degrees effectuates a rotation of the shaft portion 202 and the attached end effector about 90 degrees or about 180 degrees. In aspects, a clutch (not shown) disengages lever arm 202 from the gear system at the second position of the lever arm 202 such that the lever arm 202 may be returned to the starting position, e.g., under a bias, without altering the rotational position of the shaft portion 204. Upon returning to the starting position, the lever arm 202 is re-engaged with the gear system. A suitable push-push mechanism (including clutch and gearing system, suitably modified to provide a rotational output rather than a longitudinal output) suitable for this purpose is described in U.S. Patent No. 9,877,777, entitled "SURGICAL INSTRUMENT HAVING A BIPOLAR END EFFECTOR ASSEMBLY AND A DEPLOYABLE MONOPOLAR ASSEMBLY".

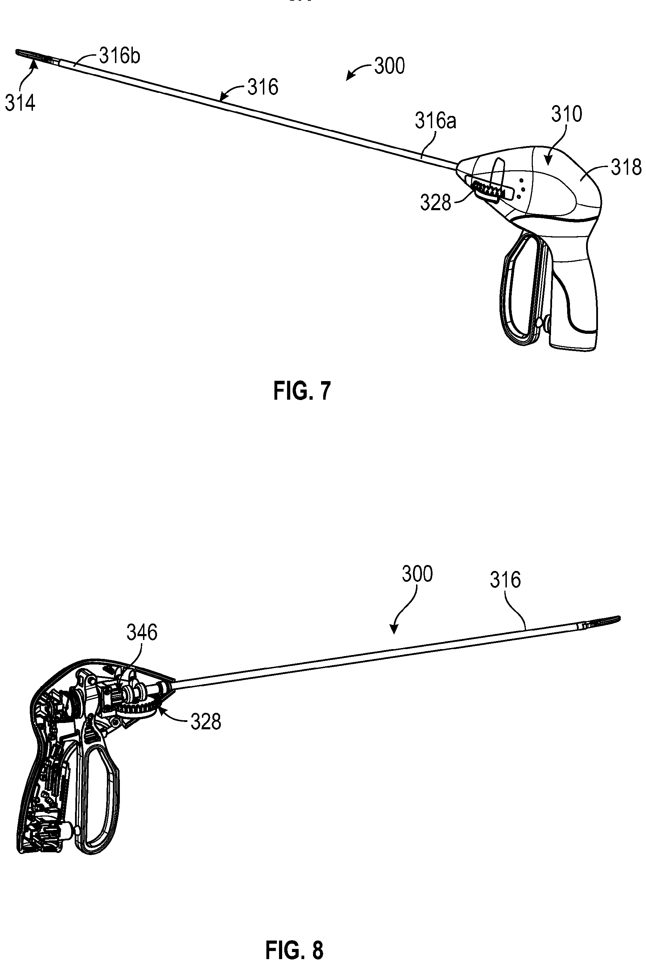

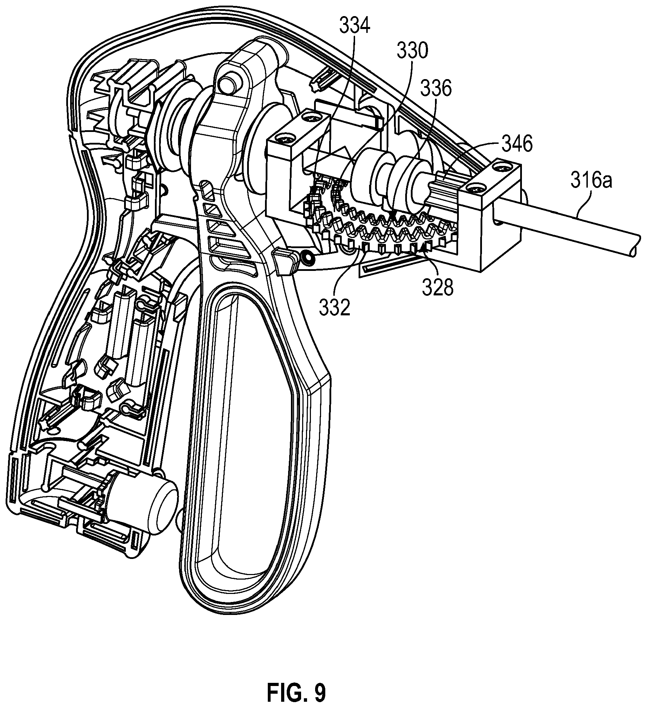

With reference to FIGS. 7-9, another electrosurgical forceps 300, similar to electrosurgical forceps 100, is illustrated, and includes a handle assembly 310, a shaft portion 316, and an end effector 314. The handle assembly 310 includes a handle housing 318, an actuator, such as, for example, a rotation wheel 328, rotatably coupled to the handle housing 318, and a main gear 346 (e.g., a pinion gear) operably coupled to the rotation wheel 328. The shaft portion 316 has a proximal end portion 316a rotatably supported by the handle housing 318, and a distal end portion 316b configured to support the end effector 314. The main gear 346 is rotationally fixed about the proximal end portion 316a of the shaft portion 316 such that the shaft portion 316 rotates relative to the handle housing 318 about a longitudinal axis defined by the shaft portion 316 in response to a rotation of the main gear 346.

The rotation wheel 328 protrudes outwardly from the handle housing 318 (e.g., through a slot defined therein on either side thereof) and lies on a plane that is parallel with the longitudinal axis of the shaft portion 316 such that the rotation wheel 328 is configured to rotate about a rotation axis that is perpendicular to the longitudinal axis of the shaft portion 316. The rotation wheel 328 has an annular upper surface 330 and an annular outer peripheral surface 332 from which a first annular array of teeth 334 extend upwardly. The first array of teeth 334 may protrude from the outer peripheral surface 332 and are in meshing engagement with the main gear 346.

The rotation wheel 328 has a larger diameter than the main gear 346 such that a full rotation of the rotation wheel 328 produces at least about two rotations of the main gear 346. More specifically, the first annular array of teeth 334 are disposed at a radial distance from the rotation axis of the rotation wheel 328 that is at least twice the diameter of the main gear 346. In some aspects, a gear ratio between the rotation wheel 328 and the main gear 346 is from about 1:5 to about 1:2.

In aspects, the rotation wheel 328 may have a second annular array of teeth 336 disposed radially inward of the first array of teeth 334. The main gear 346 may be configured for selective engagement with the first or second array of teeth 334, 336 to adjust a gear ratio between the rotation wheel 328 and the main gear 346. Since the second array of teeth 336 are radially closer to a rotation axis of the rotation wheel 328 than is the first array of teeth 334, when the main gear 346 is engaged to the second array of teeth 336, a rotation of the rotation wheel 328 rotates the main gear 346 to a lesser extent than the same amount of rotation of the rotation wheel 328 when the first array of teeth 334 of the rotation wheel 328 is engaged to the main gear 346. It is contemplated that the rotation wheel 328 may be slid relative to the main gear 346 to selectively engage the first or second array of teeth 334, 336 to the main gear 346. Similarly as above, the gear ratio may be selected such that a maximum single-finger rotation of the rotation wheel 328 provides a rotation of the shaft portion 316 of about 90 degrees or about 180 degrees.

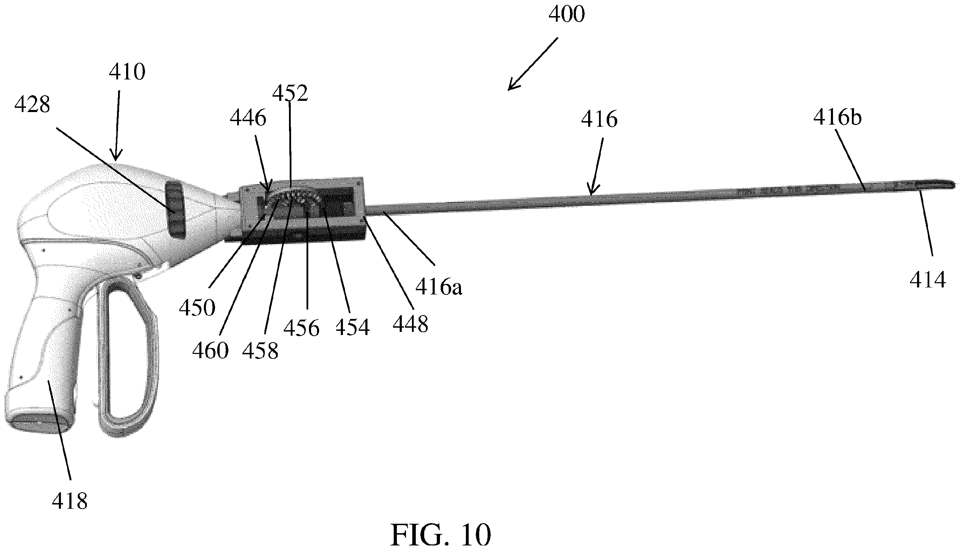

With reference to FIG. 10, another electrosurgical forceps 400, similar to electrosurgical forceps 300, is illustrated, and includes a handle assembly 410, a shaft portion 416, and an end effector 414. The handle assembly 410 includes a handle housing 418, an actuator, such as, for example, a rotation wheel 428, rotatably coupled to the handle housing 418, and a gear assembly 446 operably coupled to the rotation wheel 428. The shaft portion 416 has a proximal end portion 416a operably coupled to the gear assembly 446, and a distal end portion 416b configured to support the end effector 414.

The gear assembly 446 is housed within a bracket or housing 448 that is fixed to the handle housing 418. The gear assembly 446 is operably coupled between the rotation wheel 428 and the proximal end portion 416a of the shaft portion 416 such that a rotation of the rotation wheel 428 cases a rotation of the shaft portion 416. The gear assembly 446 includes a proximal shaft 450, a transmission wheel 452, and a distal gear 454. The proximal shaft 450 is non-rotatably coupled to the rotation wheel 428 such that the proximal shaft 450 rotates with and by the rotation wheel 428. The proximal shaft 450 has a proximal gear 456, such as, for example, a crown gear, fixed thereabout. In aspects, the proximal shaft 450 may have any suitable type of gear fixed thereabout.

The transmission wheel 452 is rotationally supported in the housing 448 and includes a lower surface defining a first set of teeth 458 arranged in an annular row, and a second set of teeth 460 arranged in an annular row. The first set of teeth 458 are disposed concentrically within the second set of teeth 460. The first set of teeth 458 are in meshing engagement with the proximal gear 456 of the proximal shaft 450 such that the transmission wheel 452 rotates in response to a rotation of the proximal shaft 450. The distal gear 454, which is fixed to the proximal end portion 416a of the shaft portion 416, may be a crown gear, and is in meshing engagement with the second set of teeth 460 of the transmission wheel 452. Since the second set of teeth 460 has a larger diameter than the first set of teeth 458, the transmission wheel 452 increases the gear ratio between the proximal gear 456 and the distal gear 454 from about 1:2 to about 1:3. It is contemplated that the distal gear 454 may be fixed to the proximal end portion 416a of the shaft portion 416 via any suitable fastening engagement, such as, for example, a clip, adhesive, or the like.

It should be understood that various aspects disclosed herein may be combined in different combinations than the combinations specifically presented in the description and accompanying drawings. It should also be understood that, depending on the example, certain acts or events of any of the processes or methods described herein may be performed in a different sequence, may be added, merged, or left out altogether (e.g., all described acts or events may not be necessary to carry out the techniques). In addition, while certain aspects of this disclosure are described as being performed by a single module or unit for purposes of clarity, it should be understood that the techniques of this disclosure may be performed by a combination of units or modules associated with, for example, a medical device.

## Claims

1. A surgical instrument, comprising:
a handle assembly including:
a handle housing (118);
a rotation wheel (128) rotationally supported in the handle housing;
a ring gear (142) rotationally fixed to the handle housing;
a plurality of planet gears (144) rotationally supported on the rotation wheel and in meshing engagement with the ring gear; and
a sun gear (146) in meshing engagement with the plurality of planet gears such that a rotation of the rotation wheel in a first direction rotates the plurality of planet gears relative to the ring gear to rotate the sun gear; and
a shaft portion (116) having a proximal end portion rotatably supported by the handle assembly, and a distal end portion configured to support an end effector (114), the shaft portion configured to rotate relative to the handle assembly about a longitudinal axis defined by the shaft portion, the shaft portion being non-rotatably coupled to the sun gear such that the shaft portion is configured to rotate in the first direction with the sun gear in response to a rotation of the rotation wheel.

2. The surgical instrument according to claim 1, wherein the sun gear is fixed to the proximal end portion of the shaft portion.

3. The surgical instrument according to claim 1 or 2, wherein the rotation wheel is configured to slide relative to the sun gear between a first position, in which the rotation wheel is engaged to the plurality of planet gears, and a second position, in which the rotation wheel is engaged directly to the sun gear.

4. The surgical instrument according to claim 1, 2 or 3, wherein a gear ratio between the rotation wheel and the sun gear is from 1:5 to 1:2.

5. The surgical instrument according to any preceding claim, wherein the actuator is a rotation wheel that protrudes outwardly from the handle housing.

## Patentansprüche

1. Chirurgisches Instrument, umfassend:
eine Griffbaugruppe aufweisend:
ein Griffgehäuse (118);
ein Drehrad (128), das drehbar in dem Griffgehäuse gelagert ist;
einen Zahnkranz (142), der drehfest an dem Griffgehäuse angebracht ist;
eine Vielzahl von Planetenrädern (144), die drehbar an dem Drehrad gelagert sind und sich in Zahneingriff mit dem Zahnkranz befinden; und
ein Sonnenrad (146) in Zahneingriff mit der Vielzahl von Planetenrädern, so dass eine Drehung des Drehrads in eine erste Richtung die Vielzahl von Planetenrädern relativ zu dem Zahnkranz dreht, um das Sonnenrad zu drehen; und
einen Schaftteil (116) mit einem proximalen Endteil, der drehbar von der Griffbaugruppe getragen wird, und einem distalen Endteil, der zum Tragen eines Endeffektors (114) gestaltet ist,
wobei der Schafteil dafür gestaltet ist, sich um eine von dem Schaftteil definierte Längsachse relativ zu der Griffbaugruppe zu drehen, wobei der Schaftteil nichtdrehbar an das Sonnenrad gekoppelt ist, so dass der Schaftteil dafür gestaltet ist, sich in Antwort auf eine Drehung des Drehrads mit dem Sonnenrad in die erste Richtung zu drehen.

2. Chirurgisches Instrument gemäß Anspruch 1, wobei das Sonnenrad an dem proximalen Endteil des Schaftteils befestigt ist.

3. Chirurgisches Instrument gemäß Anspruch 1 oder 2, wobei das Drehrad dafür gestaltet ist, relativ zu dem Sonnenrad zwischen einer ersten Position, in der das Drehrad in Eingriff mit der Vielzahl von Planetenrädern steht, und einer zweiten Position, in der das Drehrad direkt mit dem Sonnenrad in Eingriff steht, zu gleiten.

4. Chirurgisches Instrument gemäß Anspruch 1, 2 oder 3, wobei ein Übersetzungsverhältnis zwischen dem Drehrad und dem Sonnenrad von 1:5 bis 1:2 beträgt.

5. Chirurgisches Instrument gemäß einem der vorstehenden Ansprüche, wobei der Aktor ein Drehrad ist, das von dem Griffgehäuse nach außen vorsteht.

## Revendications

1. Instrument chirurgical, comprenant :
un ensemble poignée comportant :
un logement de poignée (118) ;
une roue de rotation (128) supportée en rotation dans le logement de poignée ;
une couronne dentée (142) fixée en rotation au logement de poignée ;
une pluralité de satellites (144) supportés en rotation sur la roue de rotation et en prise par engrènement avec la couronne dentée ; et
un planétaire (146) en prise par engrènement avec la pluralité de satellites de telle sorte qu'une rotation de la roue de rotation dans une première direction fasse tourner la pluralité de satellites par rapport à la couronne dentée pour faire tourner le planétaire ; et
une partie arbre (116) présentant une partie extrémité proximale supportée de manière rotative par l'ensemble poignée, et une partie extrémité distale configurée pour supporter un effecteur terminal (114), la partie arbre étant configurée pour tourner par rapport à l'ensemble poignée autour d'un axe longitudinal défini par la partie arbre, la partie arbre étant accouplée de manière non rotative au planétaire de telle sorte que la partie arbre soit configurée pour tourner dans la première direction avec le planétaire en réponse à une rotation de la roue de rotation.

2. Instrument chirurgical selon la revendication 1, dans lequel le planétaire est fixé à la partie extrémité proximale de la partie arbre.

3. Instrument chirurgical selon la revendication 1 ou 2, dans lequel la roue de rotation est configurée pour glisser par rapport au planétaire entre une première position, dans laquelle la roue de rotation est en prise avec la pluralité de satellites, et une seconde position, dans laquelle la roue de rotation est en prise directement avec le planétaire.

4. Instrument chirurgical selon la revendication 1, 2 ou 3, dans lequel un rapport d'engrenages entre la roue de rotation et le planétaire est de 1:5 à 1:2.

5. Instrument chirurgical selon une quelconque revendication précédente, dans lequel l'actionneur est une roue de rotation qui dépasse vers l'extérieur à partir du logement de poignée.
